Europäisches Patentamt

European Patent Office

Office européen des brevets

(19)

(11) Veröffentlichungsnummer : **0 049 862**
**B1**

(12) **EUROPÄISCHE PATENTSCHRIFT**

(45) Veröffentlichungstag der Patentschrift :
**15.05.85**

(21) Anmeldenummer : **81108024.1**

(22) Anmeldetag : **07.10.81**

(51) Int. Cl.⁴ : **G 01 N  3/24**, G 01 N 19/04

(54) **Verfahren und Prüfkörper zur Bestimmung der Glashaftung von Glasverbund-Zwischenschichten im Zugscherversuch.**

(30) Priorität : **11.10.80 DE 3038449**

(43) Veröffentlichungstag der Anmeldung :
**21.04.82 Patentblatt 82/16**

(45) Bekanntmachung des Hinweises auf die Patenterteilung : **15.05.85 Patentblatt 85/20**

(84) Benannte Vertragsstaaten :
**AT BE CH DE FR GB IT LI LU NL SE**

(56) Entgegenhaltungen :
**DE-A- 2 435 068**
**DE-C-   697 999**
**US-A- 4 010 641**

(73) Patentinhaber : **HOECHST AKTIENGESELLSCHAFT**
**Postfach 80 03 20**
**D-6230 Frankfurt am Main 80 (DE)**

(72) Erfinder : **Fabian, Klaus**
**Drosselweg 11**
**D-6239 Kriftel (DE)**

EP 0 049 862 B1

**0 049 862**

**Beschreibung**

Die Erfindung bezieht sich auf ein Verfahren zur Bestimmung der Glashaftung von Glasverbund-Zwischenschichten im Zugscherversuch und einen Prüfkörper, der zur Durchführung dieses Verfahrens geeignet ist.

Glasverbunde bestehen üblicherweise aus mehreren Glasplatten gleicher oder unterschiedlicher Dicke, die durch eine klebende Zwischenschicht miteinander verbunden sind. Diese Zwischenschicht besthet häufig aus einem thermoplastischen Kunststoff, insbesondere Polyvinylbutyral (PVB), der meist in Form einer Folie zum Verbinden der Glaspatten eingesetzt wird. Solche Glasverbunde werden hauptsächlich als Sicherheitsglas in der Automobil- und Bauindustrie benötigt. Die Zwischenschicht soll im Falle der Schlageinwirkung auf den Glasverbund das Loslösen von Splittern aus dem Verbund verhindern. Die Haftung des Glases an der Zwischenschicht darf jedoch nicht zu stark sein, damit die Zwischenschicht an der Stelle der Schlageinwirkung nicht überdehnt, zerrissen und durchbrochen wird. Es ist daher von Bedeutung, die Haftfestigkeit solcher Zwischenschichten exakt messen zu können ; dazu bedarf es einer genauen und möglichst einfach durchführbaren Meßmethode, mit deren Hilfe objektive Meßwerte reproduzierbar ermittelt werden können. Es ist Aufgabe dieser Erfindung, eine solche Meßmethode bereitzustellen.

Obwohl Verbundglas schon sehr lange breite Anwendung, insbesondere im Automobilbau, findet, gab es bisher jedoch keine Methode, um die Haftung der Zwischenschicht an den Glasscheiben genau zu messen. Vielmehr mußte zu Verfahren gegriffen werden, die mehr oder weniger empirisch lediglich das Verhalten von Verbundgläsern bei Schlageinwirkung ermitteln, aber keine genaue Aussage über die Haftung der Zwischenschicht zulassen. Derartige Aussagen sollten zwar mit Zugscheruntersuchungen erhältlich sein. Es fehlte aber ein Probekörper, der eine Messung möglichst praxisgerecht erlaubt hätte, vor allem auch an Verbundscheiben aus der Fertigung.

Die Einarbeitung von Nuten in den Probekörper, wie aus der DE-Offenlegungsschrift 2 435 068 bekannt, verbietet sich bei Verbundgläsern. Die Zwischenschicht aus Polyvinylbutyral, die üblicherweise verwendet wird, wird durch die beim Einfräsen solcher Nuten entstehende Wärme in nicht vorhersehbarer und nicht reproduzierbarer Weise verändert, so daß die Meßergebnisse nicht mehr aussagekräftig sind. Vermeidet man. die Erwärmung durch Zugabe von Kühlmitteln, so greifen diese die Zwischenschicht ebenfalls an, und die Messungen werden verfälscht.

Die vorliegende Erfindung löst das Problem auf eine verblüffend einfache Weise, indem statt der sonst gebräuchlichen Nuten einfach wie vom Glasschneiden bekannt die Probekörper angeritzt und stumpf gebrochen werden. Dabei bleibt die Zwischenschicht intakt, und man erhält mit derartigen Probekörpern nach dem angegebenen Verfahren aussagekräftige Meßergebnisse.

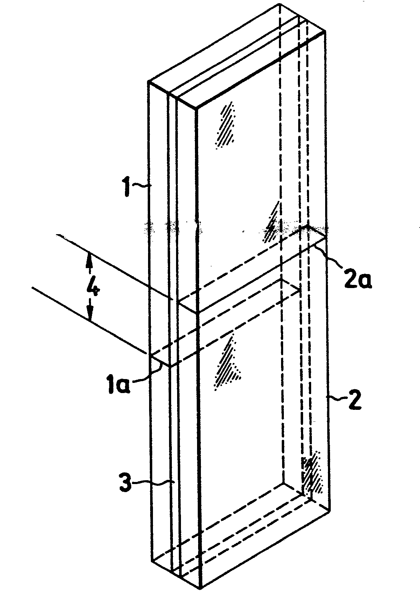

Die Erfindung betrifft nun ein Verfahren zur Bestimmung der Glashaftung von Glasverbund-Zwischenschichten im Zugscherversuch, dadurch gekennzeichnet, daß ein im wesentlichen quaderförmiger Prüfkörper (vgl. Figur 1) aus einem aus zwei Glasscheiben (1, 2) und einer Zwischenschicht (3) bestehenden Glasverbund, wobei zuvor die Glasplatten jeweils durch Ritzen und Brechen unter Bildung eines stumpfen Bruchs (1a, 2a) ohne Verletzung der Zwischenschicht geteilt und die der Zwischenschicht zugewandten Kanten der Brüche versetzt angeordnet werden, einer Zugbeanspruchung unterzogen und die zum Ablösen der Glasscheiben von der Zwischenschicht des Glasverbundes erforderliche Mindestkraft bestimmt wird.

Das Verfahren wird bevorzugt in der Weise durchgeführt, daß die Zugbeanspruchung quer zu den Brüchen (1a, 2a), die parallel zueinander und senkrecht zur Längsrichtung des Prüfkörpers angeordnet sind, gerichtet wird.

Besonders bevorzugt wird das Verfahren an einem Prüfkörper, bei dem zuvor die Brüche senkrecht zur Längsrichtung des Prüfkörpers und parallel zueinander angeordnet werden durchgeführt.

Die Erfindung betrifft ferner einen Prüfkörper zur Bestimmung der Glashaftung von Glasverbund-Zwischenschichten im Zugscherversuch, dadurch gekennzeichnet, daß der Prüfkörper aus zwei durch eine Zwischenschicht (3) deckungsgleich miteinander verbundenen rechteckigen Glasplatten (1, 2) mit jeweils einer Länge von 30 bis 100 mm, einer Breite von 5 bis 25 mm und einer Dicke von 2 bis 10 mm besteht, die jeweils durch Ritzen und Brechen unter Bildung eines stumpfen Bruchs (1a, 2a) ohne Verletzung der Zwischenschicht geteilt sind, wobei die Brüche senkrecht zur Längsrichtung des Prüfkörpers um eine Abstand (4) versetzt und parallel zueinander angeordnet sind.

Die Glasplatten des im wesentlichen quaderförmigen Prüfkörpers haben vorzugsweise jeweils eine Länge von 40 bis 60 mm, eine Breite von 10 bis 20 mm und eine Dicke von 5 bis 7 mm.

Die Fläche, die von den Längsseiten der einzelnen Glasplatten und den die Zwischenschicht berührenden Kanten der Brüche begrenzt ist, wird als Scherfläche bezeichnet. Die Größe der Scherfläche kann bei vorgegebener Breite des Prüfkörpers durch Veränderung des Abstandes der Bruchkanten voneinander variiert werden, wobei der Abstand 3 bis 20 mm, vorzugsweise 4 bis 10 mm, beträgt. Die Scherflächengröße beträgt normalerweise 0,5 bis 2,5 cm². Es ist empfehlenswert, die Größe der Scherfläche auf die zu erwartende Stärke der Scherfestigkeit abzustimmen ; bei geringer Scherfestigkeit, d. h. bei einer Scherfestigkeit von weniger als 2 MPa, ist eine Scherfläche von 1,0 bis 2,2 cm² angebracht, während bei einer mittleren Scherfestigkeit, d. h. bei einer Scherfestigkeit von 2 bis 10 MPa, eine

2

Scherfläche von 0,7 bis 1,0 cm$^2$ und bei sehr hoher Scherfestigkeit, d. h. bei einer Scherfestigkeit von mehr als 10 MPa, eine Scherfläche von 0,5 bis 0,8 cm$^2$ von Vorteil ist.

Das erfindungsgemäße Verfahren wird vorzugsweise mit den erfindungsgemäßen Prüfkörpern durchgeführt. Diese können aus einem ebenen Glasverbund oder auch aus einem einfach oder sphärisch gebogenen Glasverbund geschnitten sein, z. B. aus Automobil-Frontscheiben oder Teilstücken von Automobil-Frontscheiben ; in diesem Fall besteht die Glasverbund-Zwischenschicht vorzugsweise aus einer Polyvinylbutyral-Folie, deren Dicke vorzugsweise im Bereich von 0,1 bis 1,5 mm liegt. Mögliche Verfälschungen der Meßwerte bei der Prüfung von gebogenen Glasverbunden lassen sich durch Einsatz von Prüfkörpern mit möglichst kleinen Abmessungen weitgehend vermeiden.

In Figur 1 ist ein erfindungsgemäßer Prüfkörper dargestellt. Dabei sind die rechteckigen Glasplatten 1 und 2 durch eine Zwischenschicht 3 miteinander verbunden. Die Glasplatten 1 und 2 sind unter Bildung der stumpfen Brüche 1a und 2a geteilt, und die Brüche sind jeweils senkrecht zur Längsrichtung des Prüfkörpers um den Abstand 4 versetzt und parallel zueinander angeordnet.

Die Prüfkörper werden dadurch hergestellt, daß auf der einen Seite eines Glasverbundes mit einem üblichen Glasschneidegerät parallele Schnitte angebracht werden, deren Abstand der gewünschten Prüfkörper-Länge entspricht ; dann werden parallel zu diesen Schnitten weitere Schnitte angebracht, wodurch eine Begrenzung der Scherfläche festgelegt wird, und schließlich werden rechtwinklig zu den vorhandenen Schnitten wiederum parallele Schnitte angebracht, deren Abstand der gewünschten Prüfkörper-Breite entspricht. Nach Ausführung der Schnitte wird der Bruch der Glasplatte in folgender Weise durchgeführt : Zunächst wird das Glas an den Schnitten gebrochen, die die Länge der Prüfkörper festlegen, dann an den Schnitten, die die Breite der Prüfkörper festlegen, und schließlich an den Schnitten, die der Scherflächenbegrenzung dienen. Die andere Seite des Glasverbundes wird entsprechend behandelt, so daß quaderförmige Prüfkörper entstehen, die aus zwei durch eine Zwischenschicht deckungsgleich miteinander verbundenen, rechteckigen Glasplatten bestehen, die jeweils unter Bildung eines stumpfen Bruches senkrecht zur Längsrichtung des Prüfkörpers geteilt sind.

Das erfindungsgemäße Verfahren wird vorzugsweise mit Hilfe eines Meßgerätes durchgeführt, das zur Prüfung von Zugscherfestigkeiten verschiedener Materialien geeignet ist. Es ist vorteilhaft, wenn dieses Meßgerät mit einer Schreibeinrichtung versehen ist, die die Meßwerte automatisch aufzeichnet. Es ist empfehlenswert, als Meßgerät eine Zugprüfmaschine gemäß DIN 51 221 Teil 3 zu verwenden.

Der Prüfkörper wird mit Hilfe von Einspannvorrichtungen im Meßgerät befestigt, vorzugsweise durch Spannbacken, die eine einwandfreie Übertragung der Zugbeanspruchung vom Meßgerät auf den Prüfkörper in Richtung der Prüfkörper-Längsachse gewährleisten. Dazu sind die Einspannvorrichtung vorzugsweise über Kugelschalen und Kreuzgelenke mit dem Meßgerät verbunden, so daß etwaige Dreh- und Pendelbewegungen während der Messung ausgeglichen werden. Der Prüfkörper wird beim Einspannen in der Mittelachse der Einspannvorrichtungen ausgerichtet ; er muß rutschfest in den Einspannvorrichtungen fixiert werden. Der Prüfkörper wird zweckmäßigerweise so eingespannt, daß die Teile der Glasplatten, die die Scherfläche bedecken, von den Einspannvorrichtungen nicht erfaßt werden.

Das erfindungsgemäße Verfahren zeichnet sich dadurch aus, daß es mit einfach und schnell herstellbaren Prüfkörpern und unter Einsatz herkömmlicher Meßgeräte durchgeführt wird und eine objektive Beurteilung der Proben aufgrund gemessener und errechneter Werte erlaubt. Die Meßwertstreuung liegt bei einer Serie von 20 Messungen zwischen 5 und 10 Prozent.

Die folgenden Beispiele dienen zur näheren Erläuterung der Erfindung.

Dabei wird durch Division der maschinell gemessenen Bruchlast (N) durch die Scherfläche (mm$^2$) die Zugscherfestigkeit (MPa) errechnet.

Beispiel 1

Aus einer sphärisch gebogenen, asymmetrischen Verbundglasscheibe werden 20 annähernd quaderförmige Prüfkörper mit jeweils einer Länge von etwa 50 mm und einer Breite von etwa 15 mm geschnitten. Die Schnitte werden so durchgeführt, daß die schwächste Scheibenkrümmung in der Längsrichtung der Prüfkörper verläuft. Der Glasverbund besteht aus zwei Glasplatten mit einer Dicke von 3,0 mm bzw. 2,0 mm, die durch eine 0,76 mm dicke Schicht aus handelsüblichem Polyvinylbutyral, das 20 Gewichtsprozent Vinylalkoholeinheiten und 2 Gewichtsprozent Vinylacetateinheiten aufweist, miteinander verbunden sind. Die beiden Glasplatten jedes Prüfkörpers sind im Mittelteil stumpf gebrochen ; die Brüche verlaufen parallel zueinander und senkrecht zur Längsrichtung des jeweiligen Prüfkörpers, und die der Zwischenschicht zugewandten Kanten der Brüche verlaufen im Abstand von etwa 7 mm. An jedem Prüfkörper wird die Scherfläche ausgemessen. Jeder Prüfkörper wird dann einer Zugscherfestigkeitsprüfung mit Hilfe einer Zugprüfmaschine gemäß DIN 51 221 Teil 3 unterworfen, und aus der jeweils gemessenen Bruchlast wird die Zugscherfestigkeit errechnet. Einzelheiten sind aus Tabelle 1 ersichtlich.

(Siehe Tabelle 1 Seite 4 f.)

Tabelle 1

| Probe Nr. | Scherfläche Länge (mm) | Breite (mm) | Bruchlast (N) | Zugscher-festigkeit (MPa) |
|---|---|---|---|---|
| 1 | 14,5 | 6,7 | 198 | 2,04 |
| 2 | 14,6 | 6,9 | 215 | 2,13 |
| 3 | 15,0 | 7,0 | 234 | 2,23 |
| 4 | 15,1 | 6,9 | 226 | 2,17 |
| 5 | 14,6 | 7,0 | 204 | 2,00 |
| 6 | 13,8 | 7,1 | 241 | 2,46 |
| 7 | 14,2 | 6,9 | 232 | 2,37 |
| 8 | 15,3 | 7,0 | 247 | 2,31 |
| 9 | 14,0 | 7,0 | 233 | 2,28 |
| 10 | 14,7 | 7,0 | 236 | 2,29 |
| 11 | 14,5 | 7,0 | 218 | 2,15 |
| 12 | 13,5 | 6,8 | 208 | 2,23 |
| 13 | 14,4 | 6,6 | 235 | 2,47 |
| 14 | 14,9 | 6,7 | 228 | 2,28 |
| 15 | 13,7 | 6,8 | 217 | 2,33 |
| 16 | 15,5 | 6,6 | 218 | 2,13 |
| 17 | 14,2 | 6,7 | 217 | 2,28 |
| 18 | 14,3 | 6,4 | 193 | 2,11 |
| 19 | 14,8 | 6,5 | 217 | 2,26 |
| 20 | 14,1 | 6,4 | 190 | 2,11 |
| Mittelwert: | | | | 2,23 |

Beispiel 2

Beispiel 1 wird wiederholt unter Einsatz einer spärisch gebogenen, symmetrischen Verbundglasscheibe, deren Einzelscheiben jeweils eine Dicke von 3,0 mm aufweisen ; die Zusammensetzung des Zwischenschichtmaterials ist unbekannt. Der Abstand der Bruchkanten beträgt etwa 6 mm. Einzelheiten der Zugscherfestigkeitsprüfung sind aus Tabelle 2 ersichtlich.

Tabelle 2

| Probe Nr. | Scherfläche Länge (mm) | Breite (mm) | Bruchlast (N) | Zugscher-festigkeit (MPa) |
|---|---|---|---|---|
| 1 | 14,8 | 5,8 | 130 | 1,51 |
| 2 | 14,1 | 5,8 | 123 | 1,50 |
| 3 | 14,8 | 5,7 | 139 | 1,65 |
| 4 | 14,7 | 5,5 | 125 | 1,55 |
| 5 | 14,3 | 5,6 | 133 | 1,66 |

Tabelle 2 (Fortsetzung)

| Probe Nr. | Scherfläche Länge (mm) | Breite (mm) | Bruchlast (N) | Zugscher-festigkeit (MPa) |
|---|---|---|---|---|
| 6 | 14,4 | 5,5 | 132 | 1,67 |
| 7 | 14,6 | 5,5 | 137 | 1,71 |
| 8 | 15,0 | 5,6 | 134 | 1,60 |
| 9 | 14,5 | 5,6 | 137 | 1,69 |
| 10 | 14,8 | 5,8 | 143 | 1,67 |
| 11 | 14,0 | 5,9 | 137 | 1,66 |
| 12 | 15,1 | 6,4 | 162 | 1,68 |
| 13 | 14,5 | 6,5 | 160 | 1,70 |
| 14 | 15,4 | 6,5 | 166 | 1,77 |
| 15 | 14,8 | 6,6 | 160 | 1,64 |
| 16 | 13,8 | 6,5 | 154 | 1,72 |
| 17 | 14,7 | 6,3 | 141 | 1,52 |
| 18 | 15,0 | 6,3 | 164 | 1,74 |
| 19 | 14,3 | 6,2 | 153 | 1,73 |
| 20 | 14,9 | 6,2 | 147 | 1,59 |
| Mittelwert | | | | 1,65 |

## Beispiel 3

Beispiel 1 wird wiederholt unter Einsatz einer ebenen, symmetrischen Verbundglasscheibe, deren Einzelscheiben jeweils eine Dicke von 3,0 mm aufweisen ; die Zusammensetzung des Zwischenschichtmaterials ist unbekannt. Der Abstand der Bruchkanten beträgt etwa 5 mm. Einzelheiten der Zugscherfestigkeitsprüfung sind aus Tabelle 3 ersichtlich.

Tabelle 3

| Probe Nr. | Scherfläche Länge (mm) | Breite (mm) | Bruchlast (N) | Zugscher-festigkeit (MPa) |
|---|---|---|---|---|
| 1 | 14,6 | 5,0 | 376 | 5,15 |
| 2 | 15,1 | 5,2 | 393 | 5,00 |
| 3 | 14,4 | 5,0 | 360 | 5,00 |
| 4 | 15,0 | 5,1 | 370 | 4,84 |
| 5 | 14,8 | 5,2 | 380 | 4,93 |
| 6 | 14,9 | 5,1 | 374 | 4,92 |
| 7 | 14,9 | 4,9 | 457 | 4,89 |
| 8 | 14,5 | 5,0 | 335 | 4,62 |
| 9 | 14,8 | 5,0 | 365 | 4,93 |
| 10 | 14,9 | 5,1 | 375 | 4,93 |
| 11 | 14,3 | 5,0 | 360 | 5,04 |

Tabelle 3 (Fortsetzung)

| Probe Nr. | Scherfläche Länge (mm) | Breite (mm) | Bruchlast (N) | Zugscher- festigkeit (MPa) |
|---|---|---|---|---|
| 12 | 14,8 | 4,9 | 371 | 5,12 |
| 13 | 14,9 | 5,1 | 400 | 5,26 |
| 14 | 14,6 | 4,9 | 356 | 4,98 |
| 15 | 14,8 | 5,1 | 361 | 4,78 |
| 16 | 14,3 | 4,7 | 326 | 4,85 |
| 17 | 14,7 | 4,9 | 341 | 4,73 |
| 18 | 14,3 | 5,1 | 340 | 4,66 |
| 19 | 15,0 | 4,8 | 367 | 5,10 |
| 20 | 15,2 | 4,8 | 354 | 4,85 |
| Mittelwert: | | | | 4,93 |

Beispiel 4

Beispiel 1 wird wiederholt unter Einsatz einer ebenen, symmetrischen Verbundglasscheibe, deren Einzelscheiben jeweils eine Dicke von 3,0 mm aufweisen ; die Zusammensetzung des Zwischenschichtmaterials ist unbekannt. Der Abstand der Bruchkanten beträgt etwa 5 bis 6 mm. Einzelheiten der Zugscherfestigkeitsprüfung sind aus Tabelle 4 ersichtlich.

Tabelle 4

| Probe Nr. | Scherfläche Länge (mm) | Breite (mm) | Bruchlast (N) | Zugscher- festigkeit (MPa) |
|---|---|---|---|---|
| 1 | 15,0 | 4,7 | 565 | 8,01 |
| 2 | 14,5 | 5,0 | 510 | 7,03 |
| 3 | 14,6 | 5,0 | 515 | 7,05 |
| 4 | 14,7 | 4,8 | 470 | 6,66 |
| 5 | 14,6 | 4,5 | 540 | 8,22 |
| 6 | 14,5 | 4,5 | 500 | 7,66 |
| 7 | 14,5 | 4,8 | 475 | 6,82 |
| 8 | 14,8 | 5,1 | 515 | 6,83 |
| 9 | 14,6 | 4,6 | 440 | 6,55 |
| 10 | 14,6 | 4,8 | 395 | 5,64 |
| 11 | 14,8 | 4,5 | 450 | 6,76 |
| 12 | 14,9 | 4,4 | 395 | 6,03 |
| 13 | 14,7 | 4,6 | 420 | 6,21 |
| 14 | 14,7 | 4,7 | 425 | 6,15 |
| 15 | 14,3 | 5,6 | 495 | 6,18 |
| 16 | 14,5 | 5,7 | 595 | 7,20 |
| 17 | 14,2 | 5,6 | 580 | 7,24 |
| 18 | 14,6 | 6,1 | 630 | 7,07 |
| 19 | 14,8 | 6,0 | 690 | 7,77 |
| 20 | 14,5 | 6,4 | 640 | 6,90 |
| Mittelwert: | | | | 6,90 |

## Beispiel 5

Beispiel 1 wird wiederholt unter Einsatz einer ebenen, symmetrischen Verbundglasscheibe, deren Einzelscheiben jeweils eine Dicke von 3,0 mm aufweisen ; die Zusammensetzung des Zwischenschichtmaterials ist unbekannt. Der Abstand der Bruchkanten beträgt etwa 6 mm. Einzelheiten der Zugscherfestigkeitsprüfung sind aus Tabelle 5 ersichtlich.

Tabelle 5

| Probe Nr. | Scherfläche Länge (mm) | Breite (mm) | Bruchlast (N) | Zugscherfestigkeit (MPa) |
|---|---|---|---|---|
| 1 | 16,1 | 6,0 | 1050 | 10,87 |
| 2 | 14,8 | 5,7 | 1050 | 11,82 |
| 3 | 15,0 | 6,0 | 1000 | 11,11 |
| 4 | 14,9 | 5,9 | 995 | 11,32 |
| 5 | 14,7 | 6,0 | 1085 | 12,30 |
| 6 | 14,8 | 5,9 | 1105 | 12,65 |
| 7 | 15,0 | 5,8 | 1095 | 12,69 |
| 8 | 14,8 | 5,8 | 875 | 10,19 |
| 9 | 15,1 | 5,9 | 1080 | 12,12 |
| 10 | 14,5 | 5,9 | 985 | 11,51 |
| 11 | 14,7 | 6,0 | 1030 | 11,68 |
| 12 | 14,4 | 6,0 | 1020 | 11,81 |
| 13 | 14,5 | 5,9 | 990 | 11,57 |
| 14 | 14,6 | 5,8 | 865 | 10,21 |
| 15 | 15,2 | 5,8 | 1085 | 12,31 |
| 16 | 14,8 | 5,9 | 1115 | 12,77 |
| Mittelwert: | | | | 11,7 |

**Patentansprüche**

1. Verfahren zur Bestimmung der Glashaftung von Glasverbund-Zwischenschichten im Zugscherversuch, dadurch gekennzeichnet, daß ein im wesentlichen quaderförmiger Prüfkörper aus einem aus zwei Glasscheiben (1, 2) und einer Zwischenschicht (3) bestehenden Glasverbund, wobei zuvor die Glasplatten jeweils durch Ritzen und Brechen unter Bildung eines stumpfen Bruchs (1a, 2a) ohne Verletzung der Zwischenschicht geteilt und die der Zwischenschicht zugewandten Kanten der Brüche versetzt angeordnet werden, einer Zugbeanspruchung unterzogen und die zum Ablösen der Glasscheiben von der Zwischenschicht des Glasverbundes erforderliche Mindestkraft bestimmt wird.

2. Verfahren nach Anspruch 1, dadurch gekennzeichnet, daß die Zugbeanspruchung quer zu den Brüchen (1a, 2a), die parallel zueinander und senkrecht zur Längsrichtung des Prüfkörpers angeordnet sind, gerichtet wird.

3. Verfahren nach Anspruch 1, dadurch gekennzeichnet, daß es an einem Prüfkörper, bei dem zuvor die Brüche senkrecht zur Längsrichtung des Prüfkörpers und parallel zueinander angeordnet werden, durchgeführt wird.

4. Prüfkörper zur Bestimmung der Glashaftung von Glasverbund-Zwischenschichten im Zugscherversuch, dadurch gekennzeichnet, daß der Prüfkörper aus zwei durch eine Zwischenschicht (3) deckungsgleich miteinander verbundenen rechteckigen Glasplatten (1, 2) mit jeweils einer Länge von 30 bis 100 mm, einer Breite von 5 bis 25 mm und einer Dicke von 2 bis 10 mm besteht, die jeweils durch Ritzen und Brechen unter Bildung eines stumpfen Bruchs (1a, 2a) ohne Verletzung der Zwischenschicht geteilt sind, wobei die Brüche senkrecht zur Längsrichtung des Prüfkörpers um einen Abstand (4) versetzt und parallel zueinander angeordnet sind.

**0 049 862**

## Claims

1. A process for determining the adhesion to glass of interlayers for laminated glass by the tensile shear test, which comprises subjecting to a tensile strain a substantially parallelepiped-like test specimen of laminated glass consisting of two sheets of glass (1, 2) and of one interlayer (3), each of said sheets of glass is before divided by scratching and breaking without damaging the interlayer to form an obtuse fracture (1a, 2a), the edges of the fractures facing the interlayer being staggered, and determining the minimum force required to detach the interlayer from the glass sheets of the glass laminate.

2. A process as claimed in claim 1, which comprises effecting the tensile strain transversely to the fractures (1a, 2a) which latter are arranged parallel to one another and vertically to the longitudinal axis of the test specimen.

3. A process as claimed in claim 1, which comprises employing the process to a test specimen in which the fractures are before arranged vertically to the longitudinal direction of the test specimen and parallel to one another.

4. A test specimen for determining the adhesion to glass of interlayers for laminated glass by the tensile shear test, which comprises a test specimen consisting of two rectangular sheets of glass (1, 2) of from 30 to 100 mm length each, of from 5 to 25 mm width each and of from 2 to 10 mm thickness each, said sheets of glass being congruently linked together by an interlayer (3) and said sheets of glass being divided by scratching and breaking without damaging the interlayer to form an obtuse fracture each (1a, 2a) and said fractures being arranged vertically to the longitudinal direction of the test specimen and parallel to one another and staggered by a certain distance (4).

## Revendications

1. Procédé pour déterminer, dans un essai de cisaillement sous traction, l'adhérence au verre de couches intermédiaires de stratifiés en verre, procédé caractérisé en ce qu'on soumet à une contrainte de traction une éprouvette essentiellemet parallélipipédique, formée en un stratifié en verre consistant en deux panneaux de verre (1, 2) et en une couche intermédiaire (3), les panneaux de verre ayant été au préalable subdivisés par entaille et cassure, avec formation d'une cassure émoussée et plane (1a, 2a) sans endommagement de la couche intermédiaire, et les bords de la cassure tournés vers la couche intermédiaire étant décalés, et l'on détermine la force minimale nécessaire pour séparer les panneaux de verre de la couche intermédiaire du stratifié en verre.

2. Procédé selon la revendication 1, caractérisé en ce que la contrainte de traction est dirigée transversalement aux cassures (1a, 2a), lesquelles sont parallèles entre elles et perpendiculaires à la direction longitudinale de l'éprouvette.

3. Procédé selon la revendication 1, caractérisé en ce qu'il est mis en œuvre sur une éprouvette dans laquelle les cassures ont été au préalable disposées parallèlement entre elles et perpendiculairement à la direction longitudinale de l'éprouvette.

4. Eprouvette pour la détermination, dans un essai de cisaillement sous traction, de l'adhérence au verre de couches intermédiaires de stratifiés en verre, éprouvette caractérisée en ce qu'elle consiste en deux plaques de verre (1, 2) rectangulaires, reliées l'une à l'autre par une couche intermédiaire (3) qui les recouvre de manière égale, les plaques ayant chacune une longueur de 30 à 100 mm, une largeur de 5 à 25 mm et une épaisseur de 2 à 10 mm, et étant chacune subdivisée, par entaillage et cassure en formant à chaque fois une cassure (1a, 2a) émoussée plane sans endommagement de la couche intermédiaire, les cassures étant disposées de manière à être parallèles entre elles et à être décalées d'une distance (4) perpendiculairement à la direction longitudinale de l'éprouvette.

8